# EUROPEAN PATENT APPLICATION

(11) **EP 0 923 922 A2**
(43) Date of publication of application: **23.06.1999**
(21) Application number: 98310331.8
(22) Date of filing: 16.12.1998
(51) Int. Cl.: A61G 13/02, A61B 6/04

(54) **Improved operating room table**

(30) Priority: 18.12.1997 US 993262
(71) Applicant: Stille-Beta, Inc., Akron, Ohio 44311 (US)
(72) Inventor: Bradcovich, James Martin, Akron, Ohio 44301 (US)
(74) Representative: Spall, Christopher John

(57) **Abstract**

Operating room table suitable for both surgical procedures and digital imaging procedures, and for Minimally Invasive Surgical procedures requiring real time imaging. The table (20) of this invention comprises a radiolucent table top (22), a support structure (24) for the table top, and a base (26) for supporting the table top and the support structure. The table top is capable of vertical lift, longitudinal tilt, lateral tilt, and four-way floating movement in either a longitudinal or lateral direction, or both. The operating room table is preferably mobile. The table further comprises normally locked first and second locks (150, 152), one for preventing longitudinal floating movement of the table top and the other for preventing lateral floating movement of the table top. the table further comprises a control switch or actuator (38) for releasing the locks. This control switch when actuated is effective to permit floating movement of the table top along a horizontal axis and ineffective to permit floating movement along a tilted axis. Thus, the table top is prevented from floating movement when tilted both longitudinally and laterally, is free to float laterally but not longitudinally when tilted only longitudinally, is free to float longitudinally but not laterally when tilted only laterally, and is free to float in either direction when horizontal.

## Description

### Technical Field

This invention relates to operating room tables, and particularly to an improved operating room table which is suitable for carrying out minimally invasive surgical (MIS) procedures.

### Background

Non-invasive and minimally invasive surgical (MIS) techniques are gaining widespread acceptance for many operations. These techniques are beginning to be used for high risk operations such as open heart surgery (see article in March 3, 1997 issue of *Newsweek* Magazine).

Two examples will illustrate the difference between traditional operating techniques and new minimally invasive surgical techniques.

The first illustration is open heart surgery. Traditionally, the surgeon would "crack open" the patient's chest, breaking ribs and perhaps even removing one or more ribs in order to gain access to the heart. Such procedure is painful and results in a long, slow recovery. Minimally invasive surgical procedures, on the other hand, require a much smaller opening, do not require the surgeon to "crack open" the chest or to remove ribs, and result in much more rapid recovery by the patient.

Another example is repair of an aortic aneurism. Conventionally, the surgeon would make a long vertical incision in the chest of the patient, extending from just below the neck to the abdominal region. Then the surgeon would resect the damaged portion of the aorta, which would have a "bubble" where the aorta wall is enlarged and correspondingly thinner, and therefore liable to burst at any time, and would replace the damaged portion of the aorta with nylon tubing. New minimally invasive procedures permit the surgeon to make a small incision below the pelvis, exposing an artery in the patient's leg, and to use a balloon catheter with a "stent," which is a slotted stainless steel tube for repair of the damaged portion of the aorta. This procedure is much less risky than the conventional procedure, and has a shorter recovery time.

Patient support tables for medical procedures have developed along two separate and quite different lines. Operating tables form the first line or group. Imaging tables for medical examination and diagnostic imaging form the second group.

Current operating room tables are designed primarily for "visual" surgeries. A visual surgery is a surgical procedure in which the surgeon is looking directly at the organ on which he or she is operating, and relying on previously taken still images, such as x-rays, MRI's, CT Scans or other fixed frame diagnostic information. Now with the advent of non-invasive and minimally invasive surgical techniques, the surgeon is viewing an image of the organ in real time rather than the organ itself.

Currently available operating room tables are not sufficient to meet the demands created by non-invasive and minimally invasive surgical techniques. Traditional operating room tables may be either fixed or mobile. Such a table is typically capable of being raised or lowered, is also typically capable of longitudinal tilt to either a head-low or head-high position (Trendelenberg and Reverse Trendelenburg, respectively), and may also be capable of lateral tilt. The table top on which the patient is supported may have the capacity for longitudinal extensibility. Capability of other movements of the table top, including lateral movement, is typically lacking. Also, the table top typically has a table top with a high degree of x-ray radiation absorption, resting on a mattress pad of equal or greater x-ray absorption, and supported in a structural frame which further results in scattering of radiation, e.g., x-rays or other types of radiation used for diagnostic purposes. A typical operating room table top will have an absorbtivity equivalent to that of a layer of aluminum 1.5 mm thick, and the mattress pad may have equal of greater x-ray absorption, giving a total absorbtivity equivalent to that of a layer of aluminum 3mm or more. Resulting image quality is poor.

A traditional operating room table for conventional surgery required little or no imaging capabilities. X-ray imaging is normally not carried out during the course of a conventional surgical operation, because radiation doses are so high that medical personnel (surgeons and technicians) would not be in the operating room when x-ray imaging was being done, and because x-ray films typically take some time (say 30 to 40 minutes) to develop. Diagnostic imaging was done in advance of the operation, and typically at another location. X-ray imaging would also be carried out after an operation was complete as a checking procedure. In either case, medical personnel are not in the room where x-rays are being taken.

Poor resolution quality, high radiation dosages which require everyone except the patient to leave the room while x-rays are being taken, and a finite time required for developing an x-ray film, all preclude the use of x-rays using a conventional operating room table for real time imaging.

Another shortcoming of conventional operating room tables is that they are quite difficult to move about. Sometimes such movement is required during the course of surgery. The surgeon would then give a command to an assistant, frequently having to shout because of operating room noise in order to be heard. An assistant would move the table to position the patient for x-ray. Many times it is necessary to move the patient to avoid metal structure in the table top. Since conventional operating room tables are difficult to move about, the assistant would be able to move the table only with difficulty and resulting lack of precision.

Diagnostic procedures for conventional surgery are carried out in a room, separate from an operating room, equipped with diagnostic equipment, such as an x-ray machine, an MRI apparatus, or a CT scan apparatus, which typically is mounted on a fixed C arm. Imaging tables used in such rooms are quite different from operating room tables. An imaging table is typically capable of imaging a patient from head to toe. This is in contrast to a conventional operating room table, which typically cannot take images over the entire length of a patient. (Head-to-toe imaging on a operating room table is seldom necessary, however; only occasionally is x-ray imaging of a patient below the knees required.) The typical imaging table may be mounted on either a fixed base or a mobile base. The imaging table typically has four-way "float," which is the ability of the table to be moved manually in either a longitudinal direction or a lateral direction from a "home" position. Such tables are typically elongated and longitudinally extending so as to support the entire length of a patient. A typical imaging table is incapable of being tilted either longitudinally or laterally. An imaging table typically has a radiolucent top which permits passage of radiation used for diagnostic purposes, e.g., x-rays or gamma rays, with relatively little resistance. These radiolucent table tops typically meet a radiation standard promulgated by the United States Food and Drug Administration (FDA) radiation standard, which basically is that the resistance to radiation offered by the table top must not exceed that equivalent to 1.0 mm of aluminum for fixed tables and 1.5 mm of aluminum for mobile tables. Four-way float capability enables a user to move the table either longitudinally or laterally with a patient thereon, so as to position the patient optimally for diagnostic radiation imaging. Such table tops are much easier to move around as needed than those used in a operating room. However, imaging table for diagnostic procedures typically lack the ruggedness of surgical operating tables.

Some patients require both heart surgery and cardiac catheterization. Under conventional procedures, a heart operation may be performed first in an operating room. Then at a later date (which typically can be anywhere from a few days to a few weeks later) the patient is taken to a catheter laboratory for angiography and/or x-rays. The latter procedures utilize an imaging table as afore described. Because of the different requirements for an operating room and for a catheter laboratory, and in particular the different requirements for the respective tables used in such rooms, different rooms using different types of tables have been utilized.

As stated earlier, the recent advent and rampant growth and use of non-invasive and minimally invasive surgical procedures requires new patient tables which possess characteristics and capabilities not possessed by either conventional operating room tables or conventional catheterization laboratory and imaging tables. Real time imaging used in MIS procedures requires high-quality images obtained at low radiation dosages. High quality images are required because the image in effect becomes the "eyes" of the surgeon. Low radiation dosages are required because the surgeon and other operating room personnel must remain in the operating room while imaging is being carried out, without exceeding safe radiation doses over a lifetime. An eight-hour MIS procedure may require as much as 90 minutes of imaging; it can be seen that low radiation dosages are indeed required. Furthermore, neither conventional operating room tables nor conventional diagnostic imaging tables possess all of the degrees of freedom of movement which are needed in a operating room table for MIS procedures.

### Objects and Summary

An object of this invention is to provide an operating room table which is suitable for both radiologic examination procedures and surgical procedures, and in particular, an operating table which is suitable for non-invasive and minimally invasive surgical procedures which require real time imaging as a surgical procedure is in progress.

This invention provides an improved operating table which is suitable for both radiologic examination procedures and surgical procedures, and which is particularly suitable for non-invasive and minimally invasive surgical procedures which require real time imaging as an operation is in progress. The operating table of this invention employs a radiolucent table top which enables a user to obtain distortion-free images with low levels of radiation. The operating table of this invention also provides vertical lift, longitudinal tilt (Trendelenburg and Reverse Trendelenburg), lateral tilt, and four-way float. The term "four-way float" denotes the capability of moving a table top either longitudinally or laterally, or both, relative to a "home" position.

An operating room table (or apparatus) according to this invention comprises a longitudinally extending radiolucent table top having a longitudinal axis and a lateral axis; a supporting structure for the table top; and a base for supporting the table top and the supporting structure. The support structure provides lift, longitudinal tilting, lateral tilting, and longitudinal and lateral axial movement of the table top. Longitudinal and lateral movement of the table top are commonly manual and in this case may be referred to as four-way floating movement or float. The table of this invention further comprises locking means for preventing longitudinal or lateral movement of the table top. The locking means comprise a first normally locked lock for preventing lateral (or y-axis) movement of the table top, and a second normally locked lock for preventing longitudinal (or x-axis) movement of the table top. The two locks are locked and released individually. The table of this invention further comprises an actuator for releasing the locks individually and thereby permitting longitudinal or lateral movement, or both. The actuator when actuated is effective to permit movement of the table top along a horizontal axis and ineffective to permit floating movement of the table top along a tilted axis. Consequently:
1. The table top is prevented from either longitudinal or lateral movement when tilted both longitudinally and laterally.
2. The table top is free to move laterally but not longitudinally when tilted only longitudinally (i.e., about a lateral or y-axis).
3. The table top is free to move longitudinally but not laterally when tilted only laterally (i.e., about a longitudinal or x-axis).
4. The table top is free to move either longitudinally or laterally when horizontal.

### Brief Description of the Drawings

In the drawings:

Fig. 1 is a top plan view of a preferred operating room table according to this invention.

Fig. 2 is a side elevational view of the table shown in Fig. 1, with parts broken away.

Fig. 3 is an end elevational view of the table shown in Fig. 1.

Fig. 4 is a schematic view of a lock or brake for preventing movement of the table top in one direction, which may be either lateral or longitudinal.

Fig. 5 is a schematic diagram of an electrical circuit for releasing the locks which normally hold the table top in place, thereby permitting lateral and/or longitudinal movement of the table top.

### Description of the Preferred Embodiments

This invention will now be described in detail with particular reference to a preferred embodiment of this invention.

Referring now to the drawings, 20 designates an operating room table according to this invention. Operating room table 20 comprises a longitudinally extending radiolucent table top 22 having a longitudinal axis and a lateral axis; a support structure 24 for the table top; and a base 26 for supporting the table top 22 and the support structure 24.

Both table top 22 and base 26 are generally rectangular and longitudinally extending. The directions of the respective longitudinal or x-axes of the table top 22 and the base 26 are parallel. Both extend in the same direction, i.e., forwardly from the support structure. Both the table top 22 and the base 26 have lateral or y-axes which are perpendicular to their respective longitudinal or x-axes. The table top 22 is supported above base 26 at a height that is adjustable and generally convenient for surgical and operating procedures in an operating room. Table top 22 comprises a cantilevered first or forward portion 22a, and a second or rearward portion 22b, which is supported by support structure 24. At least part of the second portion 22b is directly above the support structure 24. The two portions 22a and 22b are integrally formed as a unitary structure. First portion 22a is elongated and rectangular and provides an imaging area. Second portion 22b is a non-imaging area; this portion is preferrably rectangular and may be square, and may be slightly wider than the first portion 22a.

The support structure 24 extends upwardly from a rearward portion of base 26 so as to support the table top 22 in an elevated position above the base. The support structure 24 comprises a lower portion 24a and an upper portion 24b, which is a table top support assembly, above the lower portion 24a. Lower portion 24a of support structure 24 includes a lift assembly or mechanism (or lift means) for raising and lowering the table top 22. The upper portion 24b of support structure 24 includes a first tilt mechanism (or tilt means) for longitudinal tilting of the table top 22, a second tilt mechanism (or tilt means) for lateral tilting of the table top 22, and a table top mounting arrangement (or means) permitting longitudinal and lateral movement of the table top 22, all as will be described hereinafter.

The base 26, and hence the entire operating table 20, may be either fixed or mobile. A fixed base may be supported for example on a floor of a hospital room which is intended for either radiologic examination or surgical procedures, and particularly for minimally invasive surgical procedures. However, the preferred base 26, as shown, is mobile. To that end, the base 26, which is generally rectangular in shape and has four corners, may be supported on pivotal wheels or casters 28 (four in number, one at each corner of the base 26), which engage a floor or other generally horizontal supporting surface, enabling the table to be moved in any direction along this supporting surface. Rectangular shape of the base 26 is highly desirable when the base is mobile and is not important when the base is stationary.

The overall dimensions of table 20 may be as desired. For example, base 16 of table 20 may have an overall width of 31 inches and an overall length of 77 inches. The table top 22 may be slightly longer and narrower than the base, e.g., 82 inches and 25 inches wide. The table top may have a cantilevered first portion 22a which may be about 18 inches x 57 inches, and a second portion 22b which is about 25 inches square.

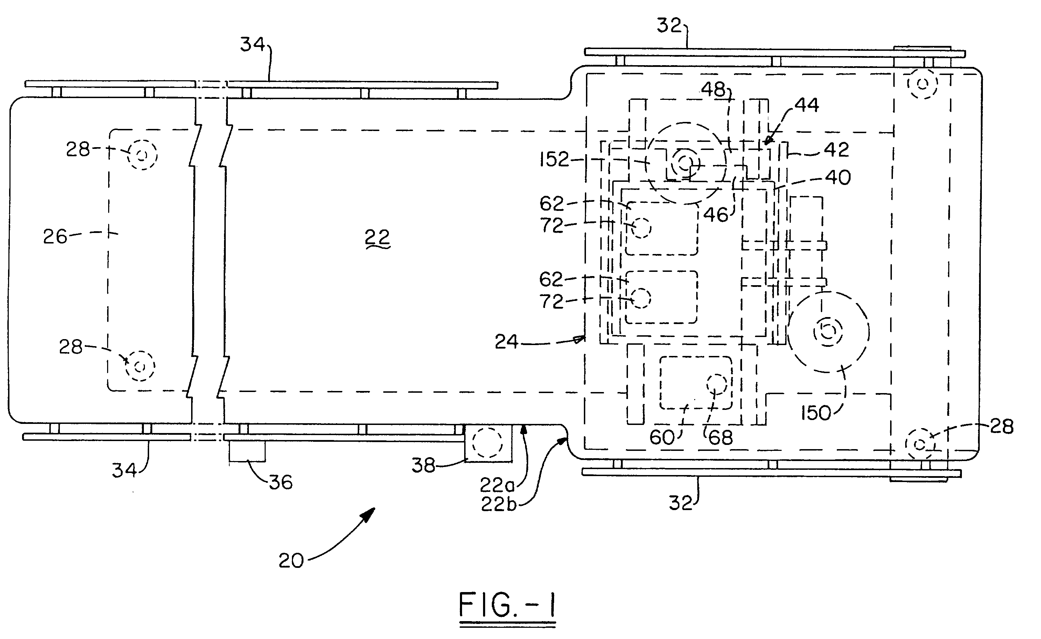

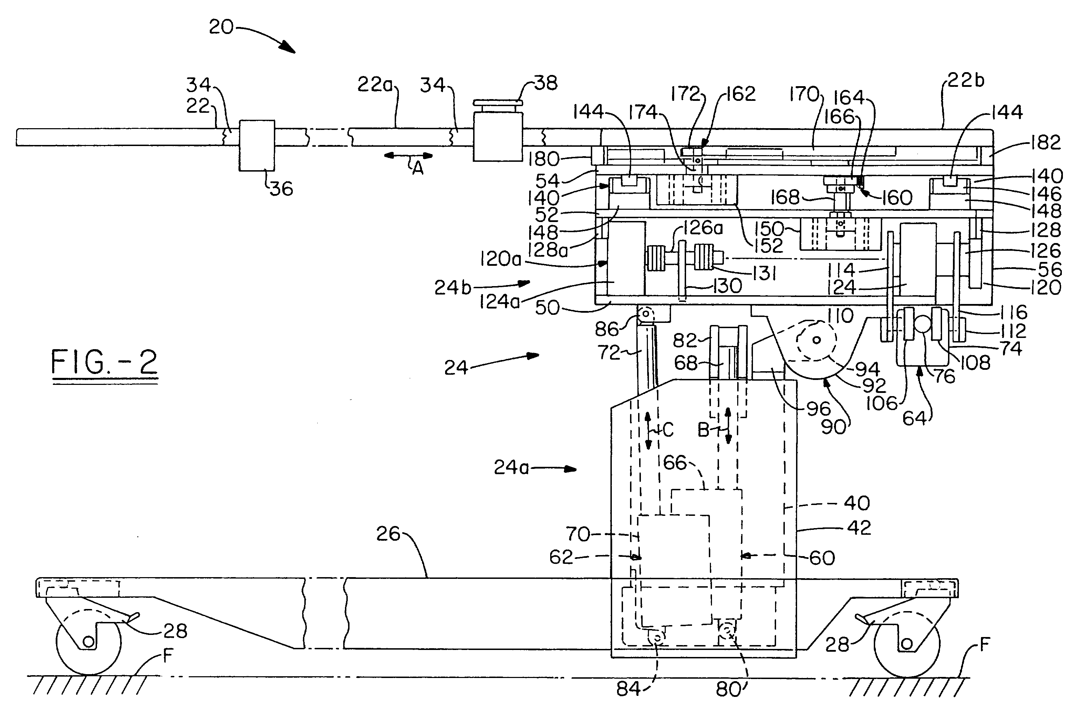

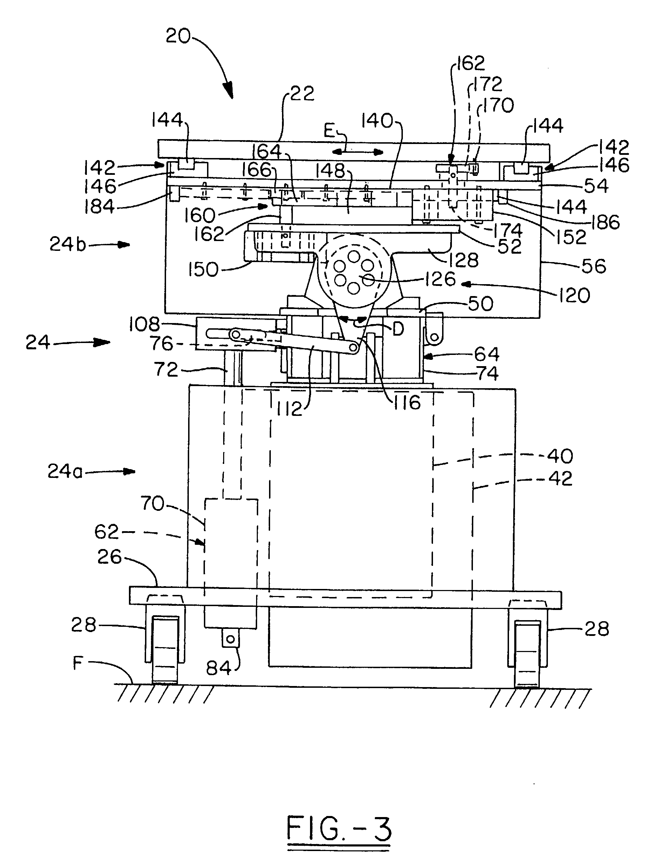

The table top 22 is shown in its normal or "home" position in Figs. 1 and 2. When the table top 22 is in its normal or "home" position, it is disposed essentially above base 26 and is horizontal. When the table top 22 is fully extended, it extends to the left as seen in Figs. 1 and 2. Movement of table top 22 between its normal position and an extended position is indicated by arrow A in Fig. 2. The height of table top 22 may be adjustable from 29 inches to 42 inches. The longitudinal travel of table top 22 may be 15.75 inches, and the lateral travel may be ±5 inches. The home position of table top 22 shown in Figs. 1 and 2 represents the limit of longitudinal travel in a rightward direction as seen in these figures. On the other hand, the table top 22 can travel laterally either to the right or to the left of the home position as seen in Fig. 3. These dimensions are merely representative and may be varied as desired.

Table 20 of this invention is stable, and therefore not inclined to tip over, both when empty and when supporting a load (i.e., a patient) weighing up to 140 kilograms (310 pounds), preferably up to 230 kilograms (500 pounds), and whether the table top 22 is in its normal position (shown in Figs. 1 and 2) or in an extended position (to the left as shown in Figs. 1 and 2). The weight distribution of table 20 is such as to achieve stability. To achieve stability of table 20, both when empty and when supporting a patient, a major amount of the empty table 20, i.e., about two-thirds to three-quarters of the total weight, is concentrated in base 26. Also, base 26 and the parts which make up the support structure 24 are rigid. Also, the casters 28 which support the base 26 on a floor F are placed essentially at the four corners of the base. As a result, whether the table 20 is empty or is supporting a patient weighing up to about 230 kilograms (500 pounds), and whether the table top 22 is in its normal position shown in Figs. 1 and 2 or is fully extended longitudinally (to the left as seen in Figs. 1 and 2), and whether the apparatus or table 20 is level from side to side (which is usually the case) or is tilted laterally up to 10° (which can occur, for example, when a table is wheeled up or down a ramp and is immediately turned to the right or left at the end of the ramp), the table 20 is stable and does not require any external support. (The maximum patient weight of 230 kilograms and the maximum lateral tilt of 10° are both preferred design limits and can be varied.)

The table top 22 is made of a radiolucent material (e.i., radiolucent to x-rays and gamma rays), preferably carbon fibers, which may be encapsulated in a suitable (and essentially radio-transparent) polymer matrix. The maximum radiation absorption of table top 22 should not exceed the radiation absorption of a sheet of aluminum 1.0 mm thick, in accordance with the current United States Food and Drug Administration (FDA) Standard (21 CFR Subchapter J). A preferred radiation absorption (of x-rays or gamma rays) is equivalent to about 0.6 to 0.7 mm of aluminum. In contrast, present operating room table tops typically have much higher radiation absorption, e.g., equivalent to about 1.5 mm of aluminum. The table top 22 should also be essentially rigid, i.e., should have a high degree of stiffness.

The table top 22 has no structural frame, since a structural metal frame will distort radiographic images. However, the table top may have thin bands of metal attached to lateral edges for supporting accessory rails 32 and 34, which are adjacent to and slightly outwardly from lateral edges of the table top at the second (or foot) portion 22b and the first (or head) portion 22a, respectively. For some purposes (e.g., for a table which is intended primarily for use in a radiological imaging room) it may be desirable to omit accessory rails 34 in the first (or imaging) portion 22a of the table top 22 while providing accessory rails 32 in the second (or non-imaging) portion 22b of the table top 22. For operating room use, including use with MIS procedures, both sets of accessory rails 32 and 34 may be provided. The head portion 22a but not the foot portion 22b is available for imaging. The head portion 22a and the foot portion 22b are so designated because a patient is normally placed on table top 22 with the head and body on head portion 22a and the legs and feet on foot portion 22b, although the patient can be placed in the opposite direction if desired. (For some procedures, the opposite direction will be preferred.)

A table 20 according to this invention may have a single radiolucent table top 22, or a set of interchangeable table tops, each of which is adapted for a particular type of surgical or diagnostic procedure. When interchangeable table tops are used, not all of the table tops in the set have to be radiolucent. For example, the set may include a non-radiolucent table top which is particularly intended for surgical procedures in which no radiographic procedures will be used.

A control console 36 and a palm button 38 may be supported on an accessory rail 34, as shown in Figs. 1 and 2. The control console 36 may contain switches for controlling vertical movement and longitudinal and lateral tilt of the table top 22. The palm button 38 provides an actuator which may be quickly and easily actuated for unlocking brakes (to be described subsequently) which prevent longitudinal and lateral manual movement of the table top 22.

The support structure 24 for table top 22 includes a vertically reciprocable carriage 40, which is preferably rectangular and tubular, and which is telescopically mounted inside a column 42, which is also preferably rectangular and tubular, and which is affixed to the base 26 and extends upwardly therefrom. Vertical movement of the carriage 40 is guided by means of a first bearing assembly (or guide means) 44, which comprises a bearing rail 46 affixed to carriage 40 and slidable in a bearing block (or bearing guide) 48, which is affixed to the column 42. The carriage 40 and column 42 are disposed in a lower portion 24a of the support structure 24.

The support structure 24 further includes a series of three spaced rectangular platforms or plates (best seen in Figs. 2 and 3), comprising a first plate 50, a second plate 52 and a third plate 54, arranged one above the other. All three plates 50, 52 and 54 reciprocate vertically with the carriage 40. The first and lowermost plate 50 can tilt longitudinally (i.e., about a lateral or y-axis) but not laterally. The second or intermediate plate 52, which is above the first plate 50, is capable of lateral tilt about an x-axis. The third and uppermost plate 54 can float laterally, affording lateral float to the table top 22. Finally, the table top 22 is mounted on the uppermost plate 54 so that it can move longitudinally relative thereto. All three plates are rectangular in shape. These three plates are all disposed above the carriage 40 and column 42, and together form an upper portion 24b of support structure 24. This upper portion 24b of support structure 24 may also be referred to as a table top support assembly. The uppermost or third plate 54 extends the entire length and width of the upper portion of support structure 24, as may be seen in Figs. 2 and 3. The first and second plates 50 and 52, respectively, may extend the entire length of this upper portion of the support structure 24, but are not as wide as the uppermost plate 54 as may be seen in Fig. 3. A platform cover 56, comprising four vertical side walls extending downwardly from respective sides of the third or uppermost plate 54, may be provided.

A series of power driven actuators, which are preferably linear actuators, control the vertical movement, longitudinal tilt and lateral tilt of table top 22. A first set of linear actuators (first actuator means) comprising a single linear actuator 60, controls vertical movement. A second set of linear actuators (second actuator means) 62, comprising a pair of linear actuators in side-by-side relationship (as best seen in Fig. 1), control longitudinal tilting of the table top 22 between a head-low (Trendelenburg) and head-high (reverse Trendelenburg) position. A third set of linear actuators (third actuator means), comprising a single linear actuator 64, controls lateral tilting of the table top 22. A harmonic drive may replace the linear actuator 64 for lateral tilt.

The linear actuators 60, 62 and 64 may be structurally similar. First linear actuator 60 includes a casing 66 (a first member) which houses a motor and a gear, and a reciprocable screw (a second member) 68, e.g., a ball screw or an Acme screw which extends outside the casing 66. The visible external portion of each screw is smooth and rod-like. The first and second members are relatively moveable. Similarly, each of the linear actuators 62 of the second set comprises a casing 70 and a screw 72 which are relatively moveable, and the linear actuator 64 of the third set comprises a casing 74 and a screw 76 which are relatively moveable.

Linear actuators 60, 62 and 64 may be either electrically or hydraulically powered. The former is preferred. Electrical power may be furnished by batteries (preferred ) or by external line current. When table 20 is mobile as shown and its linear actuators are battery powered, the table can be moved freely from place to place in a hospital or other patient facility as needed, since it requires no external support and is not tethered to any external objects such as an external power line.

Linear actuators 60, 62 and 64 may be controlled by a user through a control console 36, which as shown earlier may be mounted on one side of table top 22, e.g., by means of an accessory rail 34.

### Lift

A first or lower end of the first linear actuator 60 is pivotally attached to the base 26 by means of a pivot assembly 80, which may comprise, for example, a clevis attached (e.g., by welding) to the base 26, an ear attached (i.e., by welding) to the lower end of casing 66, and a shoulder bolt which extends through aligned bolt holes in the clevis and the ear. Similarly, a second or upper end of the first linear actuator, i.e., the end of screw 68 which is outside and remote from the casing 66, may be pivotally secured to the carriage 40 by means of a pivot assembly 82 which comprises a clevis welded to or otherwise fixedly mounted on the carriage, and a shoulder bolt which passes through aligned holes in the clevis and the upper end of the screw 68. Thus, reciprocating movement of the screw 68 -will raise and lower the carriage 40.

Actuation of linear actuator 60 to lift the screw 68 associated therewith causes the carriage 40 and the entire upper portion 24b of the table top support structure 24 comprising spaced plates 50, 52 and 54 and the table top 22 to be lifted up. Conversely, downward movement of the linear actuator screw 68 associated with linear actuator 60 lowers the entire upper portion 24b of the support structure 24 and the table top 22. Vertical reciprocation of linear actuator screw 68 is shown by arrow B in Fig. 2.

### Longitudinal Tilt

Longitudinal tilt is accomplished by means of a pair of linear actuators 62 (the second set) previously mentioned. The lower (or casing) ends of linear actuator 62 are attached to the carriage 40. The upper ends of linear actuator 62 (i.e., the upper ends of the respective screws 72) are attached to the underside of the first or lowermost platform or plate 50. Pivot assemblies 84 and 86, similar to those (80 and 82) previously described, and comprising a clevis and a shoulder bolt, may be used in each case, permitting small amounts of rotary movement.

The lowermost plate 50 of the support structure 24 is rotatably mounted on a first pivot bearing assembly (or pivot mechanism) 90, which comprises a pivot bearing (e.g., a pillow block) 92 fixedly secured to the underside of plate 50, a laterally extending pivot shaft 94, and a shaft support lever or linking member 96 attached to carriage 40, as shown in Fig. 2. Pivot shaft 94 is non-rotatably secured to the shaft support lever 96. Pivot shaft 94 is journaled for rotation in pillow block 92. The axis of this pivot shaft 94 (the pivot axis) extends horizontally and laterally, i.e., along a y-axis. The shaft support lever 96 and the pivot block 92 are rotatable relative to each other. Thus the first assembly 90 comprises a first part, i.e., pillow block 92, and a second part, i.e., pivot shaft 94 and shaft support lever 96, which are relatively rotatable. The shaft support member 96 forms a linking member linking the shaft 94 with the carriage 40.

The table top 22 and its support structure 24, including plates 50, 52 and 54 are shown in the drawings in the home position, which is a level position. Downward movement of linear actuator screws 72 associated with linear actuators 62 lowers the front end of first plate 50 and with it the entire upper portion of support structure 24 and table top 22 to a head down (Trendelenburg) position. Upward movement of the screws 72 associated with linear actuators 62 cause upward movement of the front end of plate 50 and with it the table top 22 about the axis of shaft 94 to a head high (reverse Trendelenburg) position. Vertical reciprocation (i.e., upward and downware movement) of screws 72 is shown by arrow C. This longitudinal axis of table top 22 tilts and the lateral axis remains level during longitudinal tilt.

### Lateral Tilt

Lateral tilt is controlled by means of a third actuator set comprising linear actuator 64 (Figs. 2 and 3) which includes a horizontally and laterally oriented cylindrical casing 74 (a first member) pivotally attached to the underside of first plate 50, and a screw 76 (a second member) extending generally horizontally therefrom. The outer end of screw 76 of linear actuator 64 includes a pin which slides in slots in a pair of linear slide pieces 106 and 108 (Fig. 3). A pair of link arms 110 and 112 are attached at first ends to a second end of linear actuator 64 (i.e., the outer end of screw 76), and at second ends to levers 114 and 116 for lateral tilt. Levers 114 and 116 in turn form part of a pivot bearing assembly (or second pivot mechanism 120. This screw 76 is operatively connected to pivot bearing assembly 120.

The second or intermediate plate 52 of table top support structure 24 (and more particularly the upper portion 24b thereof) is pivotally mounted for lateral tilting (i.e., tilting about a longitudinal or x-axis) by means of pivot bearing assemblies 120 and 120a. Pivot bearing assembly 120 is a drive assembly at a rearward end of plate 52. Pivot bearing assembly 120a is an idler assembly at a forward end of plate 52.

Pivot bearing assembly 120 includes a pillow block 124 (Figs. 2 and 3) which is mounted on the upper side of lowermost plate 50, and a pivot shaft 126, which is rotatably mounted in pillow block 124. Pivot shaft 126 is directly driven by and rotates with levers 114 and 116. Pivot shaft 126 is journaled for rotation in the second or rearward pillow block 124. This pivot bearing assembly 120 for lateral tilt further includes a pivot arm 128 affixed to pivot shaft 126 and to the underside of the second or intermediate plate 52 of the table top support structure 22. Levers 114 and 116, pivot shaft 126, and pivot arm 128 (collectively a second part of pivot bearing assembly 120) rotate as a unit in pillow block 124 (a first part of pivot bearing assembly 120). Rotation of pivot shaft 126 is shown by arrow D in Fig. 3. It will be seen that pivot bearing shaft 120 is a drive assembly and pivot shaft 126 is a drive shaft.

Idler pivot bearing assembly 120a includes a pillow block 124a, which is mounted on the upper side of lowermost plate 50, and an idler shaft 126a which is journaled for rotation in pillow block 124a. Pivot shafts 126 and 126a have a common horizontally and longitudinal extending axis (i.e., an x-axis) of rotation. Idler pivot bearing assembly 120a further includes a pivot arm 128a, which is mounted on the underside of intermediate plate 52. Pillow block 122a and pivot arm 128a are relatively rotatable. A shaft support 130 affixed to the upper side of the lowermost plate 50 provides support for a rearward end of idler shaft 126a. Cams 131 to regulate the limits of lateral tilt and for home positioning, are provided at the rearward end of idler shaft 126a, outward of shaft support 130.

Actuation of the linear actuator 64 so that its screw 76 moves outwardly, i.e., to the left as seen in Fig. 3, causes the right hand side of the table top support assembly 24b to be lowered and the left side (as seen in Fig. 3) of the table top support assembly 24b and the table top 22 to be raised. Conversely, inward movement of the screw associated with linear actuator 64 causes just the reverse movement, i.e., lowering of the left side of table top 22 and the table top support assembly 24b and its corresponding lifting of the right hand side of table top 22 and the table top support assembly, as best seen in Fig. 3.

While separate tilt mechanisms for affecting longitudinal tilt and lateral tilt (i.e., a first tilt means or mechanism for longitudinal tilting of table top 22 and a second tilt means or mechanism for lateral tilting of table top 22) are shown and are preferred, a single tilt mechanism for affecting both longitudinal and lateral tilt may be used if desired. Such mechanism may include a spherical bearing and a joystick for control. One linear actuator would replace the first set 60 and the second set 62 of linear actuators.

### Four-way float

Four-way float, or the ability to move table top 22 axially and manually in either a longitudinal (or x-axis) direction or a lateral (or y-axis) direction, is provided for by a first (or y-axis) pair of linear bearing assemblies 140 (Fig. 2) for controlling lateral or y-axis movement of the uppermost support plate 54, and a second (or x-axis) pair of linear bearing assemblies, 142 (Fig. 3) for controlling longitudinal or x-axis movement of the 22 relative to the uppermost support plate 54. Lateral movement of table top 22 is indicated by arrow E in Fig. 3. Longitudinal movement of table top 22 is shown by arrow A in Fig. 2 as previously noted. It will be noted that the uppermost support plate 54 and the table top 22 move laterally together to afford lateral movement of the table top 22, while longitudinal or x-axis movement of the table top 22 is relative to the uppermost support plate 54.

Each of the linear bearing assemblies 140, 142 comprises a bearing rail 144 which reciprocates in a bearing block 146, which serves as a guide. The respective rails 144 of the y-axis bearing assemblies 140 are affixed to the underside of the uppermost support plate 54, while the respective blocks or guides 146 are affixed through spacers 148 to the upper side of intermediate support plate 52. Similarly, the respective rails 144 of the x-axis bearing assemblies 142 are affixed to the underside of table top 22, while the respective blocks or guides 146 are affixed to the upper side of the uppermost support plate 54.

A first or y-axis brake 150 mounted on the underside of the intermediate plate 52, and a second or x-axis brake 152 mounted on the underside of the uppermost support plate 54, control floating movement of the table top 22 in the lateral (or y) direction and the longitudinal (or x) direction, respectively. The first brake (or lock) 150 and the second brake (or lock) 152 together form locking means for preventing floating movement of the table top. Brakes 150 and 152 may be alike. These brakes are normally locked, i.e., biased toward a locking position. These brakes may be spring set electromagnetic brakes, in which one or more springs bias the brake to a normally locked position, and an electromagnet which when energized overcomes the spring bias to release the brake. Such a brake may be referred to as a fail-safe brake, since it will be in the locked position in the event of power failure. Among the advantages of a fail-safe brake are: (1) the brakes 150 and 152 will be locked, so that table top 22 will not move, in the event of power failure; (2) normally locked brakes require less power than normally released brakes (which are released or unlocked when no power is applied and require application of power to lock the brakes) since the brakes 150 and 152 are locked most of the time and are unlocked only when it is desired to move table top 22 along either an x-axis or a y-axis; and (3) there is little or no heat dissipation problem in a fail-safe brake, while there may be a considerable heat dissipation problem in a normally unlocked brake, which would be energized most of the time.

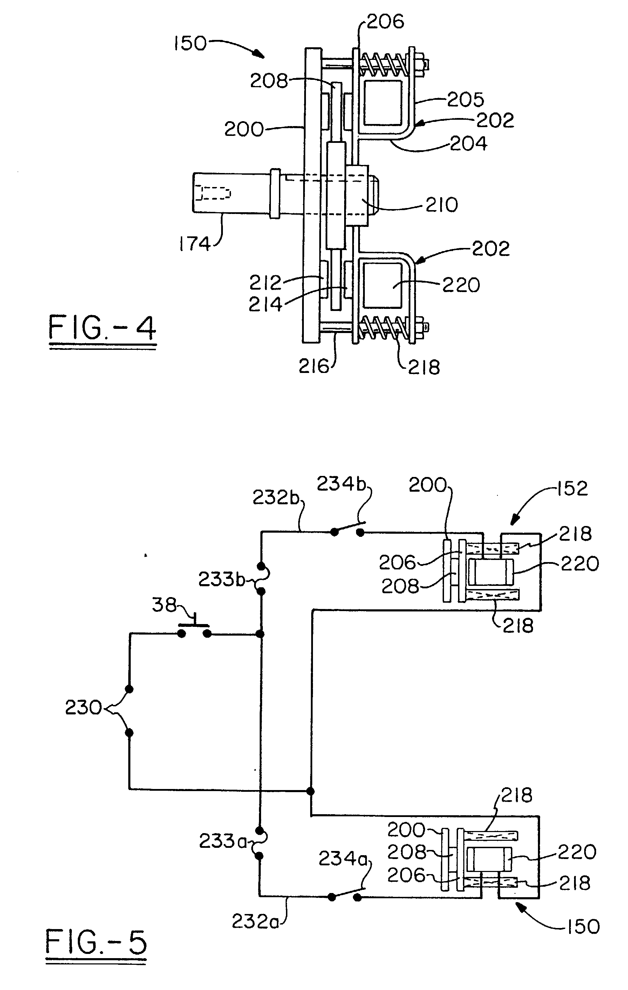

Brakes 150 and 152 and their operation will be described subsequently in greater detail with reference to Figures 4 and 5.

A preferred brake is Model No. SAB400, manufactured by Inertia Dynamics Inc. of Collinsville, Connecticut. Other suitable fail-safe brakes are obtainable from other vendors.

Brake 150 when released (by pressing palm button 38) will permit floating movement or travel of the uppermost plate 54 and table top 22 in a lateral direction when released. Similarly, the second or x-axis brake 152 will permit longitudinal movement of the table top 22 in a longitudinal direction, up to the maximum extension available, when released. When both brakes are released, a user can move the table top 22 either longitudinally or laterally, or both, manually, as desired. When the user stops moving the table top 22 and releases palm button 38, the brakes 150 and 152 will lock so as to hold the table top 22 in the position in which the user has placed it. As will be discussed in further detail, the first or y-axis brake will be releasable for lateral movement of the table top 22 only when the lateral or y-axis of the table top 22 is horizontal. Similarly, the second or x-axis brake 152 is releasable to permit longitudinal travel of the table top 22 only when the longitudinal axis of the table top 22 is horizontal.

A pair of rack and pinion gear assemblies 160 and 162 provide operative connections between brakes 150 and 152 on the one hand, and the uppermost plate 54 and table top 22, respectively, on the other. First or y-axis rack and pinon gear assembly 160 prevents lateral movement of plate 54 when brake 150 is locked or set, and permits such lateral movement when brake 150 is released. Similarly, second or x-axis rack and pinion gear 162 prevents longitudinal movement of the table top 22 when the second or x-axis brake 152 is locked or set, and permits such longitudinal movement when brake 152 is released.

The y-axis rack and pinion gear assembly 160 includes a rack 164, which extends perpendicular to the plane of the paper and a pinion shaft 168 which extends vertically to engage brake 150. Rack 164 is affixed to the uppermost plate 54. Similarly, the x-axis rack and pinion gear assembly 162 (best seen in Fig. 3) includes a rack 170, a pinion gear 172, and a vertically extending pinion shaft 174 which extends downwardly to engage the second or x-axis brake 152. The rack 170 extends perpendicular to the plane of the paper. Rack 170 is affixed to table top 22. Thus, when y-axis brake 150 is locked, the pinion 166 and the rack 164 of y-axis rack and pinion gear assembly 160 cannot move, and uppermost plate 54 is prevented from lateral movement. When y-axis brake 150 is released, uppermost support plate 54 and table top 22 can be moved manually in a lateral direction. Similarly, when the x-axis brake 152 is locked, the pinion shaft 174 and the rack 170 of x-axis rack and pinion gear assembly 162 are immobilized, and table top 22 is prevented from movement in a longitudinal direction. When x-axis brake 152 is released, the table top 22 can be moved manually in a longitudinal direction.

Longitudinal movement of the 22 is restricted to a desired range by means of x-axis end stops 180 and 182 (Fig. 2), which are engaged by bearing blocks 146 of x-axis bearing assemblies 142 as the table top moves longitudinally. Limit stops are also provided for limiting lateral movement of the 22 and the uppermost support plate 54 beyond desired limits. These may be engaged, for example, by the y-axis bearing assemblies 140.

Any desired amplitude of longitudinal and lateral movements of table top 22 may be provided. In a preferred embodiment, longitudinal movement of table top 22 from a normal or retracted position shown in the drawings to an extended position 12 inches to the left of the position shown in Fig. 2 is provided for longitudinal movement can be varied and may be of greater or less amplitude as desired. The x-axis brake or lock 152 may be capable of retaining the table top 22 in either its normal retracted position or in any extended position in which the table top 22 is moved longitudinally, or alteratively, may provide only a finite number of locked position, e.g., the normal retracted position in Fig. 2, a mid-way position six inches to the left of that seen in Fig. 2, and a fully extended position twelve inches to the left of that shown in Fig. 2. Lateral movement may be of desired amplitude, for example, plus or minus 5 inches from the centered or neutral position shown in the drawings. It will be understood that any desired degree of longitudinal or lateral movement may be provided for. While manual or floating movement of table top 22 along an x-axis and a y-axis (i.e., longitudinal movement and lateral movement, respectfully) is preferred, it will be apparent that either longitudinal movement or lateral movement, or both, of the table top 22, can be motorized if desired. One option may be to provide a motor for controlling longitudinal movement of table top 22, while relying on manual movement for affecting movement of the table top in a lateral direction. Ordinarily, it is not necessary to motorize either x-axis or y-axis movements of table top 22.

All of the motors associated with the apparatus of this invention, i.e., the motors which drive the linear actuators 60, 62 and 64, may be controlled through a single control box or switch box, which may be of the push-button type, and which may be moveably mounted on the apparatus, e.g., on a side rail of the table top. However, it is preferred to provide one control console or control box 36, which may be of the push button type, for controlling the motors of linear actuators 60, 62 and 64, and to provide a second control member actuator 38, which may be a palm button mounted on a side rail 34, for controlling release of brakes 150 and 152. A palm button switch has the advantage of quick release when a user presses or hits it with the palm of the user's hand. A palm button switch can be actuated more rapidly than a control console type switch, and does not require as much precision on the part of the user. This is very advantageous in both imaging and operating room situations, in which a radiologist or a surgeon may need to move a patient rapidly as imaging needs require. The imaging apparatus (e.g., x-ray, CT Scanner, fluoroscope or scintillation device), is typically mounted in a fixed position, as for example on a C arm, and movement of the patient relative to the imaging apparatus is accomplished by moving the table 22 on which the patient is placed relative to the imaging apparatus.

Even when longitudinal movement of table top 22 is motorized (or both longitudinal and lateral movements of table top 2 are motorized), it is desirable to provide a palm button 38 and to provide locks (which may be either brakes 150 and 152 or clutches) which can be released by actuation of palm button 38 so as to permit rapid manual movement or table top 22 along a horizontal axis (either longitudinal or lateral, or both) when the palm button 38 is actuated. This retains the emergency capability which the palm button 38 provides in tables in which all longitudinal and lateral movement of the table top 22 is effected manually.

A preferred brake 150 (or 152, since the two brakes are preferably identical) is shown schematically and described with reference to Fig. 4. Fig. 4 shows schematically a brake 150 (or 152) in a released position.

Referring now to Fig. 4, brake 150 has a generally cylindrical housing which includes a first circular (or annular) friction plate (or first housing member) 200 having a central opening therein, a second annular plate (or second housing member) 202 which may have an internally extending cylindrical portion 204, and an annular portion 205 which extends outwardly from cylindrical portion 204. The first plate 200 and the second plate 202 are spaced apart by a fixed distance.

Inside the housing are an axially moveable but non-rotatable friction plate 206 of ferromagnetic material, an annular rotary disk 208 which is between first friction plate 200 and internal friction plate 206 and in spaced relationship with both, and an annular hub 210 which is either integral with or keyed to rotate with disk 208.

Brake pads 212 and 214 (both or which are annular) are provided on an interior surface of plate 200 and on a surface of friction plate 206, respectively. Both pads face disk 208.

Hub 210 has a circular central opening which is keyed to receive pinion shaft 174 (which has an end portion that is correspondingly keyed) in driving engagement.

A series of screws 216 extend axially from the periphery of housing plate 200 to the periphery of second housing plate 202. Compression springs 218 surround portions of each of these screws. These compression springs bear against the second housing plate 202 and the reciprocable friction plate 206, urging the latter to a brake-locking position. In this brake-locking position, the brake pads 214 on reciprocable friction plate 206 are compressed against one face of rotary disk 208, compressing this disk against the brake pads 212 on housing plate 200, so that disk 208, hub 210 and pinion shaft 174 are locked together in a stationary position.

Brake 150 further includes an annular electromagnetic coil 220. Coil 220, when energized, attracts friction plate 206 against the bias of springs 218 to the brake releasing position shown in Fig. 4. In this position disk 208, hub 210 and pinion shaft 174 are free to rotate. This enables a user to move table top 22 along a lateral axis (in the case of brake 150) or a longitudinal axis (in the case of brake 152).

When electromagnetic coil 220 ceases to be energized, the brake 150 is once again moved to locking position by springs 218, which urge the friction plate 206 and brake pad 214 thereon, and rotary disk 208, into locking engagement with the brake pad 212 of plate 200, as previously described.

An electrical circuit is provided for release of brakes 150 and 152 when a user actuates palm button 38. A representative circuit is shown schematically in Fig. 5. The circuit includes terminals 230 for connection to a power source, which may be an onboard electric battery or, as shown, an external power source (e.g., 240 volts AC). The circuit further includes a conductor 232 (which may be a wire), branch conductors 232a and 232b, serving brakes 150 and 152, respectively, a pair of fuses 233a and 233b, and a pair of limit switches 234a and 234b, which are in branch circuits 232a and 232b respectively. Limit switches 234a interrupts the flow of current through branch conductor 232b whenever the lateral (or y) axis of table top 22 is tilted. Similarly, limit switch 234b interrupts current through branch conductor 232b whenever the longitudinal axis (or x-axis) of table top is tilted. In this way, brakes 150 and 152 will be released to permit movement of table top 22 along a horizontal axis, but will not be released to permit movement of table top 22 along a tilted axis, whenever a user presses palm button 38. In other words, the palm button or actuator 38 is effective to permit floating movement of the table top 22 along a horizontal axis and is ineffective to permit floating movement of the table top 22 along a tilted axis. Consequently:
1. Table top 22 is prevented from movement along either a longitudinal axis (or x-axis) or a lateral axis (or y-axis) when the table top is tilted both longitudinal and laterally.
2. The table top 22 is free to move laterally but not longitudinally when tilted only longitudinally (i.e., about a lateral axis in either Trendelenburg or reverse Trendelenburg mode).
3. The table top 22 is free to move longitudinally but not laterally when tilted only laterally (i.e., from side to side about a longitudinal axis).
4. The table top 22 is free to move either longitudinally or laterally when horizontal.

Prevention of floating movement along a tilted axis is an important safety feature. If the table top 22 were free to move along a tilted axis when the palm button 38 is actuated, the table top would move quickly in a downward direction along the tilted axis until it hits a limit stop, at which point a patient on top of the table top could be catapulted off, risking injury. On the other hand, floating movement of the table top 22 along a horizontal axis is safe, and such movement in a rapid fashion is often desirable to reposition a patient quickly for imaging. This invention permits a table top 22 to be so moved whether to table top is horizontal or is tilted only along one axis.

Table top 22 can be tilted either longitudinally or laterally, or both, while it is either in its "home" position or has been axially displaced, either longitudinally or laterally, or both, from its "home" position. It is possible to move the table top 22 to a longitudinally extended position (i.e., to the left as seen in Figs. 1 and 2) and then to tilt the table top 22 about a lateral axis, so that the first or head end 22a of the table top 22 is either lower (Trendelenburg position) or raised (reverse Trendelenburg position). Such capability may be desirable for certain imaging purposes. Limit switches may be provided to prevent collision of the table top 22 with the floor or with any other object. Such limit switches may limit the amount of longitudinal (or Trendelenburg) tilt, for example when the table top 22 is in a longitudinally extended position.

It is possible, although not necessary, to provide a table 20 in which both locks 150 and 152 are released simultaneously, and are released only when table top 22 is horizontal. This could be accomplished, for example, by providing a single electrical circuit with branches for brakes 150 and 152 instead of separate electric circuits for each brake as above described. In this case, either longitudinal tilt or lateral tilt of table top 22 would cause limit switches 234 to assume an open-circuit mode so that no circuit could flow from the actuator 38 to either brake 150 or 152 unless table top 22 is horizontal. Even in this case, it would be possible to tilt the table top 22 either longitudinally or laterally, or both, while the table top is either in its "home" position or has been axially displaced either longitudinally or laterally, or both, from its "home" position.

### Operation

To use the device of this invention, a patient may be placed on the top surface of table top 22 at any desired location in a treatment facility while the device is in its retracted position as shown in the drawings. The patient may be placed on the table with the head at either end, as desired; more typically, the patient's head will be at the forward end 22a of the apparatus (to the left as seen in Figs. 1 and 2) with the feet correspondingly toward the other (or rearward or foot end) of the apparatus. The patient may be placed on the table top at any desired location in a hospital or other treatment facility. For example, the patient may be transferred from a hospital bed to the table 22. Then the apparatus with the patient on board may be wheeled to an operating room, a diagnostic imaging room, or other patient procedure room. Alternatively, a patient may be transported on a stretcher to an operating room, a diagnostic imaging room, or other patient procedure room which has an apparatus 20 according to this invention, and transferred from the stretcher to the table 20. The former procedure is more widely used in Europe than in the United States at the present time; the latter procedure is presently preferred in the United States.

In the operating room (or other patient procedure room), a mobile table 20 of this invention with the patient thereon is placed in a desired position. The table top 22 may then be lifted to desired height. If tilting either longitudinally or laterally is desired, this may be accomplished by the tilting mechanisms described previously. Finally, the table top 22 can be moved manually either longitudinally or laterally, or both, to a precisely desired position, by releasing the locks or brakes 150 and 152 through actuation of the palm button 38. Then imaging and non-invasive procedures concurrent with imaging may take place. It will again be noted that the table top can be moved longitudinally only if the longitudinal or x-axis is horizontal, and can be moved laterally only if the lateral or y-axis is horizontal. In other words, lateral tilt of the table top 22 will not preclude longitudinal movement, nor will longitudinal tilt (Trendelenburg or reverse Trendelenburg) preclude lateral movement. The table top 22 cannot be moved in either direction if it is tilted along both axes. Similarly, the table top 22 can be moved either longitudinally or laterally, or both, by a floating movement, if it is initially horizontal or level.

The table top 22 can be tilted either longitudinally or laterally, or both, in any position, whether it is in its "home" position or has been displaced either longitudinally or laterally, or both, from its "home" position. Also, if the table top 22 has been tilted either longitudinally or laterally, or both, and it is desired to change one or both tilt angles, this can be done whether the table top is in its "home" position or has been moved either longitudinally or laterally, or both, therefrom. In other words, the table of the preferred embodiment of this invention, as shown and described herein, does not contain any lockout mechanisms which prevent actuation of either the longitudinal tilt or the lateral tilt mechanisms. If desired, however, one can provide tilt lockout mechanisms which will prevent longitudinal tilting or change of longitudinal tilt angle (i.e., tilt about a lateral or y-axis, or Trendelenburg or reverse Trendelenburg) when the lateral or y-axis is not level, and correspondingly can prevent lateral tilt (tilt about a longitudinal or x-axis) or change of lateral tilt angle when the longitudinal or x-axis is not level. However, it is not necessary or even desirable to prevent tilting in either direction just because the table top has been displaced from its home position and/or is not level.

When both longitudinal and/or lateral movement or float of the table top 22 from its home position, and tilting of the table top 22 are desired, the floating movement should be carried out first and then the table top may be tilted as desired.

When all operating and imaging procedures are complete, the table to may be returned to the starting or neutral position shown in the drawings, i.e., horizontal and in its "home" position both longitudinally and laterally. The patient may then be wheeled back to a desired location, as for example to a patient bed in a hospital.

Ability of the table top to be both moved laterally or longitudinally by four-way float and to be tilted is very useful in diagnostic imaging. This gives a user of diagnostic imaging equipment a great deal of freedom to move the table top 22 and the patient thereon into precisely a desired position.

The table of this invention can be provided with interchangeable table tops if desired.

The present apparatus is versatile and has a table top which is compatible with digital imaging and yet strong enough for use in the operating room. The apparatus for table of this invention is ordinarily designed for use by patients weighing up to 230 kilograms (500 pounds); it is evident that the apparatus can be designed for either a greater or lesser maximum load if desired. Use of a radiolucent table top which is compatible with digital imaging allows for lower radiation doses during operations. This is turn makes it possible for operating room personnel to remain in the operating room while imaging takes place, without exceeding safe dosage limits. The preferred table is mobile and stable enough to permit extension of the table top out from directly over the base. The table according to the present invention is useful in both conventional operating room settings and in diagnostic settings in which an imaging apparatus is employed. Specific procedures include radiography, angiography and cardiac catheterization. The table of this invention is of a general utility in surgical, diagnostic imaging, and examination procedures, and is by no means limited to cardiovascular procedures. A table of this invention is particularly useful for Minimally Invasive Surgical (MIS) procedures in which real time imaging is required. As an example of improved operating procedures made possible by the table of this invention, a patient may undergo a heart operation (for example, heart bypass surgery) in an operating room, and then may be taken to a catheterization laboratory for angiography and/or x-ray. Traditionally, these would be scheduled as separate procedures anywhere from a few days to several weeks apart; now they can be carried out as part of a single procedure.

Furthermore, the preferred table allows access on all sides. The table allows multiple procedures to be carried on in the same room at the same time. For example, one can bring a mobile CT scanner into an operating room. It is even possible to use different imaging modalities in the same room at the same time. A table of this invention is much more easily moved about in an operating room than are conventional operating room tables and yet are sturdy enough and stable enough for operating room. This gives control to a surgeon similar to the control enjoyed by a radiologist, who traditionally has had access to a table with four-way float but without the tilting features which the table of this invention offers. The surgeon no longer has to shout orders to another person, who then moves the table with difficulty and imprecisely because of the nature of the table. Instead, the table of this invention can be moved quickly and easily as desired by the surgeon. In short, a table according to this invention possess all of the advantages of both traditional operating room tables and traditional imaging tables. It has all of the angular motions of prior operating room tables, such as lift, lateral tilt, Trendelenburg and reverse Trendelenburg, and has added extendibility compared to traditional operating room tables, for capability of traditional radiography using a c arm. The base design allows stability to permit extension of the table top out from the base. This extension is desirable to eliminate obstacles for a c arm. The preferred table it mobile, unlike the traditional operating room table which is fixed. The mobility allows the operating room to be more versatile and to provide a unit with lower construction cost. Various other capabilities and advantages will be apparent to those skilled in the art.

While this invention has been described with reference to a preferred embodiment thereof, this description is by way of illustration and not by way of limitation.

## Claims

1. An operating room table comprising:
(a) a longitudinally extending radiolucent table top having a longitudinal axis and a lateral axis;
(b) a support structure for said table top, said support structure providing lift, longitudinal tilting, lateral tilting, and longitudinal and lateral movement of said table top;
(c) a base for supporting said table top and said support structure;
(d) locking means for preventing longitudinal and lateral movement of said table top, said locking means comprising a first normally locked lock for preventing lateral movement of said table top and a second normally locked lock for preventing longitudinal movement of said table top; and
(f) an actuator for releasing said locks individually, said actuator when actuated being effective to permit movement of said table top along a horizontal axis and ineffective to permit movement of said table top along a tilted axis,
whereby:
(1) said table top is prevented from longitudinal or lateral movement when tilted both longitudinally and laterally,
(2) said table top is free to move laterally but not longitudinally when tilted only longitudinally,
(3) said table top is free to move longitudinally but not laterally when tilted only laterally,
(4) said table top is free to move either longitudinally or laterally when horizontal.

2. An operating room table according to claim 1, wherein said table top is capable of four-way manual floating movement in either a longitudinal or a lateral direction, or both, when said table top is horizontal and both of said locks are released.

3. An operating room table according to claim 1, said table being mobile.

4. An operating room table according to claim 1 in which the radiation absorbtion does not exceed that of a sheet of aluminum 1.0 mm thick.

5. An operating room table according to claim 1 wherein said support structure includes lift means for raising and lowering said table top, first tilt means for longitudinal tilting of said table top, and second tilt means for lateral tilting of said table top.

6. An operating room table according to claim 5 wherein said lift means comprises a vertically reciprocable carriage and guide means for guiding vertical movement of said carriage, and
wherein further said support structure comprises a first pivot mechanism having a lateral pivot axis for effecting longitudinal tilt of said table top, and a second pivot mechanism having a longitudinal pivot axis for effecting lateral tilt of said table top.

7. An operating room table according to claim 6 wherein said support structure further comprises first, second and third generally horizontal and vertically spaced platforms, and wherein said first pivot mechanism includes relatively rotatable first and second parts, said first part of said first pivot mechanism being supported on an underside of said first platform and said second part of said first pivot mechanism being fixedly secured to said carriage, and wherein further said second pivot mechanism includes relatively rotatable first and second parts, said first part of said second pivot mechanism being fixedly secured to an underside of said second platform and said second part of said second pivot mechanism being mounted on an upper side of said first platform.

8. An operating room table according to claim 7, further including first, second and third power driven actuator means, said first actuator means including an actuator for controlling vertical reciprocation of said table top and said supporting structure, said second actuator means including at least one actuator for controlling longitudinal tilt of said table top, and said third actuator means including an actuator for controlling lateral tilt of said table top.

9. An operating room table according to claim 7 wherein said first platform is reciprocable with said carriage and pivotable about a lateral axis, wherein said second platform is above said first platform and vertically reciprocable therewith and is further pivotable about a longitudinal axis, and wherein said third platform is above said second platform and reciprocable therewith, said third platform being slidably mounted for lateral movement relative to second platform, and wherein said table top is slidably mounted for longitudinal movement relative to said third platform.

10. An operating room table according to claim 1, further including first and second rack and pinion assemblies, each said rack and pinion assembly comprising a rack, a pinion, and a pinion shaft which engages a lock, the pinion shaft in said first rack and pinion assembly engaging said first lock, and the pinion shaft in said rack and pinion assembly engaging said second lock,
the rack in said first rack and pinion assembly being fixedly secured to said third platform and being laterally reciprocable therewith, and the rack in said second rack and pinon assembly being fixedly secured to said table top and being longitudinally reciprocable therewith.
